# EUROPEAN PATENT APPLICATION

(11) **EP 3 001 967 A1**
(43) Date of publication of application: **06.04.2016**
(21) Application number: 15185493.2
(22) Date of filing: 16.09.2015
(51) Int. Cl.: A61B 17/80, A61C 8/00, A61B 17/064

(54) **A HEAD FOR HOLDING A NAIL FOR BONE IMPLANTOLOGY AND KIT THEREOF**

(30) Priority: 30.09.2014 IT MO20140275
(71) Applicant: C.G.M. S.P.A., 42015 Correggio (RE) (IT)
(72) Inventor: PARMIGIANI, Corrado Saverio, 42015 Correggio (Reggio Emilia) (IT)
(74) Representative: Paparo, Aldo

(57) **Abstract**

A head (10) for holding a nail (100) for bone implant applications, comprising a positioning body (20) having a first cavity (21) suitable for reversibly holding a nail (100) and a second cavity (22) opposite to and in communication with said first cavity (21), and wherein said first cavity (21) and said second cavity (22) are open outwardly, an abutment element (30) configured for interacting with the nail (100) so as to bring it from a rest position to a working position thereof; wherein the positioning body (20) is suitable for holding the nail (100) within the first cavity (21) in the rest position thereof, and wherein the positioning body (20) is configured for guiding the nail (100) and keep it aligned with respect to a direction of translation (T) of the abutment element (30) when passing from the rest position to the working position of the nail (100).

## Description

The invention relates to a head for holding a nail and nail destined for bone implantology and kit thereof.

In particular, the present invention is intended to cover a head for holding a nail and nail and kit thereof which can be advantageously used within the surgery field, particularly for bone implant applications in the orthodontist field.

Indeed, the invention has for an object a head for holding a nail provided with a very small nail (termed "micro nail"), specifically used for securing membranes, osteosynthesis plates or other "mechanical" supports which are grafted onto the patient's gingiva in order to favour bone and tissue regeneration, for example in the case of a dental implantation.

In accordance with the present invention, the kit includes a sterile package ready for use which surrounds and protects the head for holding a nail and the nail inserted therein, which kit is ready for application of the nail to the gingival bone tissue.

It shall be appreciated that the term "nail" is used in the specific jargon within the technical scope of the present invention to define an object, preferably a metal object, exhibiting a shaped head, a stem and a sharp end, which is so conformed as to be inserted into a bone tissue. Needless to say that a nail used for bone implant purposes within the orthodontist field, is much smaller than a conventional nail, for example, it has a diameter of a few tenths of a millimeter and a stem length of a few millimeters.

Application of the nail into the bone tissue generally occurs by means of a device suitable for generating an impulsive force of such intensity to overcome the resistance offered by the gingival bone tissue, thus causing the nail to penetrate until a pre-determined position is reached. For this reason, even the micro nails as well as the common nails destined for surgical use, exhibit a head which is suitable to receive the impulsive force for implantation into the tissue.

In the prior art, the nail head is preferably a flat head. In other cases the nail, due to conformation thereof, is defined by a threaded stem which acts more like a common screw than a nail to be inserted.

According to the prior art, the impulsive force needed for inserting the nail into the gingival bone tissue, is generated manually by a surgeon with the aid of a pair of tools, whereof the first one consists of a small hammer which generates the impulsive force, whilst the second one is suitable for keeping in position the nail and transmitting the impulsive force thereto, until the nail consolidates within the bone tissue.

The tool used for holding the nail is called in jargon "nails positioner". In particular, the nails positioner exhibits a spindle shape, wherein at a first end thereof, the nail to be implanted is positioned and retained in a reversible manner through a shaped portion adapted to receive at least the head of the nail. At a second end, opposite the first, there is provided a flat head which is suitable for receiving the pulse from the small hammer by impact, thereby transmitting it to said nail.

Generally, the procedure for implanting a nail into the bone tissue provides that the positioning of the nail is done manually by an experienced physician who holds it in position until the impulsive force transmitted on the nail, causes the nail's stem to penetrate the bone tissue of the patient thus becoming implanted therein. Once the insertion is completed, the shaped head of the nail only remains exposed and visible, while the stem of the nail is retained within the tissue.

In the prior art, the tools and methodology of which use is made for inserting a nail intended for bone implant applications, exhibit some drawbacks. Such drawbacks often make it difficult, long and sensitive the step of implanting a nail into the bone tissue.

One drawback is that almost all nail insertion operations are performed manually by the surgeon thereby coming to be subject to the human factor, which aspect does not enable to achieve a good repeatability of the surgical operation, but above all, it does not ensure a high quality of surgical procedures and of the intervention itself. A further drawback is the rather small size of the nail and the structure of the nails positioner which is bulky and unsuited for properly following the positioning movements of the nail performed by the experienced physician. In particular, the end of the positioner which is predisposed for retaining the nail, may sometimes be hidden from surgeon's sight, who is performing the surgery in the rear gingival areas.

Additionally, in the prior art, both surgeon's hands are occupied when he/she is making a surgical intervention: one hand holds the hammer and one hand keeps the nail positioner pointed on the patient's gingiva. Another drawback consists in the difficulty of positioning and inserting the nail with the stem axis thereof being almost perpendicular to the portion of the gingiva to be treated. A positioning or partial insertion of the nail with axis thereof extremely inclined relative to the tissue surface, results in a reduced penetration capacity of the nail into the tissue and/or in an ineffective securing thereto. A possible new nail installation is no longer repeatable in the same bone area tissue in that it was already perforated thus becoming unsuitable for the purpose.

Generally, the nail chosen is placed on a transport tray predisposed for gathering all the nails which are needed by the surgeon to perform implantation into the gingival bone. Once the nail was taken from the tray and fitted onto the positioner, the most sharp and sensitive part thereof (i.e. the tip of the stem) is basically left exposed without any protection. In this way, the nail may come in contact with non-sterile objects or with objects whose sterility degree is not compatible with end use of the nail itself, furthermore there is also the risk the surgeon and/or the patient being pricked or injured by the nail where a wrong manual operation is taken.

A further drawback is the considerable number of manual surgical operations that the surgeon has to perform for removing the nail and installing it onto relevant positioner and thereafter into the bone tissue of the patient. The greater the number of interventions the surgeon has to perform, the greater the risk of error or the risk of unforeseen events that might prejudice the outcome of the entire intervention and/or reduce quality thereof. It is an object of the present invention to overcome the drawbacks of the prior art by providing a head for holding a nail and a nail for bone implant applications and related kit not exhibiting the above drawbacks.

In particular, it is object of the present invention to provide a head for holding a nail and a nail for bone implant applications and related kit which allow to temporarily hold a nail in a pre-determined position, until the same comes to be at least partially inserted into the gingival bone tissue of a patient with a single gesture of the operator's hand.

It is a further aim of the invention to ensure that the nail can be kept in the same position and to enable insertion thereof via a repeatable and accurate surgical operation which results to be less invasive for the patient.

A further object of the present invention is to make available a head for holding a nail and a nail for bone implant applications and related kit allowing to speed up and facilitate bone implantation operations in compliance with all safety and sterility principles required according to the case.

Furthermore, the disclosed head for holding a nail and a nail for bone implant applications and related kit, enables to reduce physical and psychological stress to which the surgeon and/or the patient are subject during bone implantation surgery.

These and other objects are substantially attained by a head for holding a nail for bone implant applications and related kit according to the description of one or more of the appended claims.

Further characteristics and advantages will better emerge from the detailed description of a preferred and non-exclusive embodiment of a head for holding a nail for bone implant applications and related kit according to the present invention.

Such description is provided with reference to the accompanying figures also illustrated by way of non-limiting example, wherein:
- Figure 1 is a schematic sectional view of a head for holding a nail in accordance with the present invention, in a rest position of a nail;
- Figure 2 is a schematic sectional view of the head for holding the nail of figure 1 in an operational position of a nail;
- Figure 3 is a schematic sectional view of the kit for the application device of a nail for bone implantation, according to the present invention. With reference to Figure 1 herewith annexed, by 10 it was generally indicated a head for holding a nail 100 in a preferred and non-limiting embodiment according to the present invention. The nail 100 is schematically illustrated in the appended figures in the preferred embodiment thereof and is not to be construed as limiting the scope of the invention in any manner.

In brief, the nail 100 for bone implant applications, of which further characteristics will be better illustrated in the detailed description, exhibits a head portion 110 comprising a head surface 120, a stem 130 and a sharp end portion 140 for insertion into the bone tissue. A connection portion is preferably comprised between the head portion 110 and the stem 130, which connection portion exhibits a radiused shape with a pre-determined radius of curvature, the head portion 110 further exhibits a flared shape, as shown schematically in the appended figures.

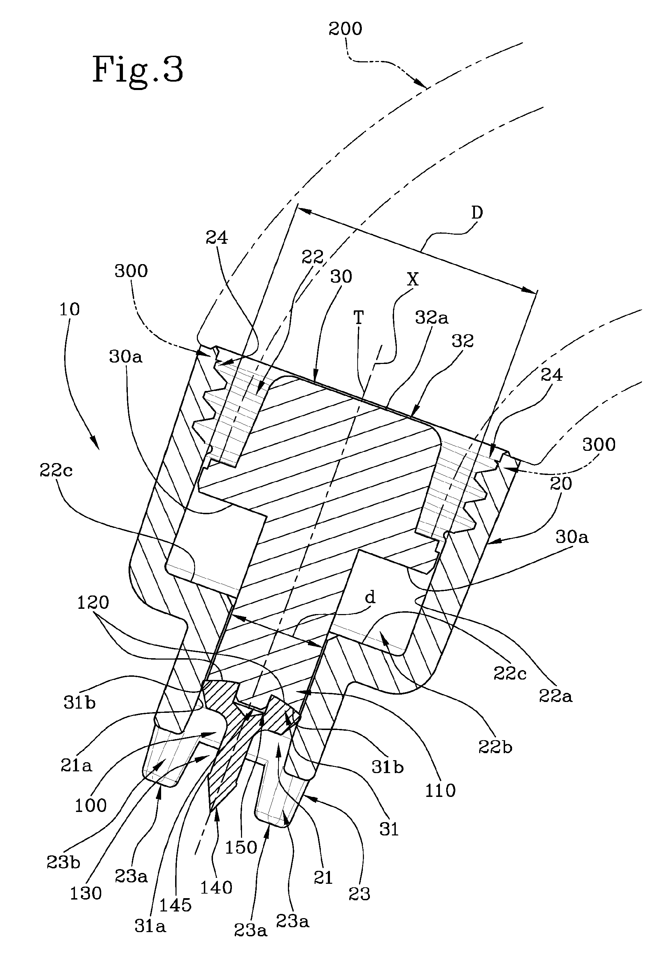

The nail 100 preferably comprises an annular portion 145 in relief between the sharp end portion 140 of said nail 100 and the stem 130, in order for the diameter relative to the end portion 140 (Figure 3) to be slightly increased.

The nail 100 in accordance with the inventive concept of the present invention comprises a stem 130 which is preferably smooth, in other words free of any helical grooves or threads, thus exhibiting a substantially cylindrical outer surface.

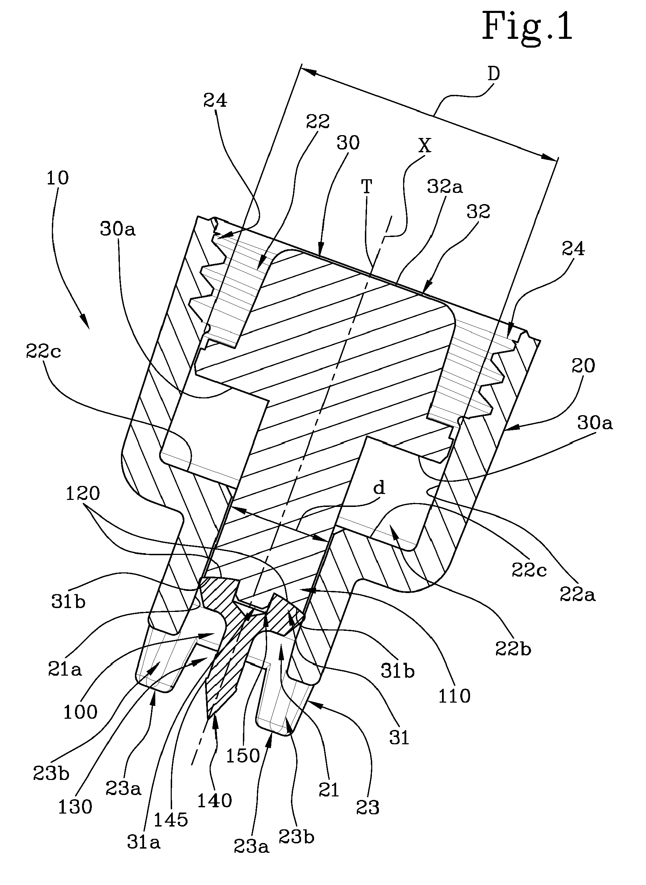

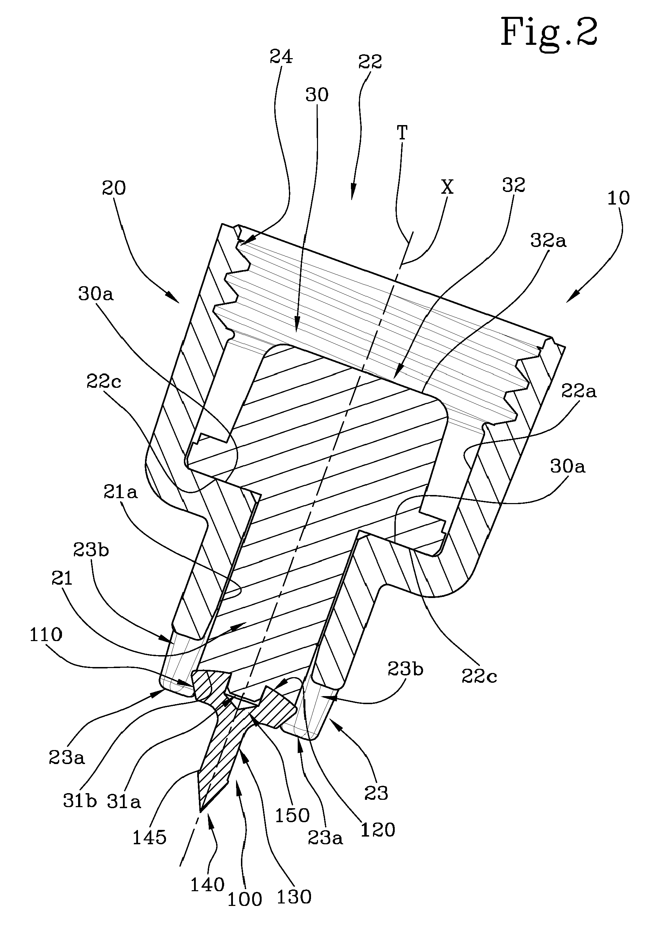

The head 10 for holding a nail 100 comprises a positioning body 20 and an abutment element 30 being operationally associated with one another. In particular, the abutment element 30 is movable relative to the positioning body 20, thereby coming to define at least two positions of the nail 100 within the head 10, i.e. a rest position and an operating position which are better described hereinafter and illustrated, by way of example, in the appended Figures 1 and 2.

Referring now to the positioning body 20, the same has a first cavity 21 suitable to reversibly hold a nail 100, and a second cavity 22 opposite the first cavity 21. The first cavity 21 and second cavity 22 are outwardly open. Further, the first cavity 21 and second cavity 22 are connected one to another.

Preferably, the positioning body 20 exhibits the first cavity 21 and second cavity 22 thereof aligned along a longitudinal axis "X" and symmetric about said axis "X".

Preferably, the first cavity 21 has a smaller cross-section than the one of the second cavity 22. In particular, with reference to the annexed Figure 1, the first cavity has a cylindrical surface 21 a having a diameter indicated by the "d". Preferably, also the second cavity 22 of the positioning body 20 has a cylindrical surface 22a having a diameter indicated by "D". Preferably, the diameter "D" of the second cavity 22 is greater than the diameter "d" of the first cavity 21, still more preferably it has a value approx. between 5 mm and 8 mm.

In an alternative embodiment, not illustrated, of the present invention, the diameter "D" of the second cavity 22 is equal to the diameter "d" of the first cavity 21 of the positioning body 20.

The diameter "d" of the first cavity 21 of the positioning body 20 is congruent to a diameter of a cross section of the nail 100, preferably with a diameter of the head portion 110 of the nail 100, as schematically illustrated by way of example in the annexed Figure 1.

In detail, by the term congruent in respect to the diameter "d" of the first cavity 21, it is meant a diameter value such as to generate a slight interference between the cylindrical surface 21 a of the first cavity 21 and the head portion 110 of the nail 100 when the latter is disposed within the first cavity 21.

The positioning body 20 comprises an annular portion 23 disposed at one open end of said first cavity 21, said annular portion being preferably afforded in a single piece with the positioning body 20. The annular portion 23 preferably defines the opening of the first cavity 21 outwardly. With respect to a transverse plane, the annular portion 23 preferably exhibits a shape corresponding to the shape of the cylindrical surface 21 a. The annular portion 23 is so arranged as to allow insertion/extraction of a nail 100 with a slight interference thereof, for example via an elastic deformation occurring relative to an outer end 23a at each passing of the nail 100. It should be appreciated that, by the term outer end 23a, it is meant a peripheral end of the cylindrical surface 21 a which is substantially circular in shape with respect to a transverse plane.

The annular portion 23 exhibits radial notches 23b, preferably along the outer end 23a. The radial notches 23b are such as to extend in depth along a direction parallel to the longitudinal axis "X", along a portion of the cylindrical surface of the first cavity 21. The radial notches 23b confer flexibility characteristics to the outer end 23a during an operational condition, wherein a nail 100 is inserted into/extracted from the head 10 for holding the nail 100.

Preferably, the outer end 23a of the annular portion 23 has an internal diameter approximately equal to the diameter "d" of the cylindrical surface 21 a of the first cavity 21.

The second cavity 22 has an annular-shaped abutment surface 22c at a bottom end 22b, which abutment surface 22c is adapted to receive a respective abutment surface 30a of the abutment element 30. In other words, the cylindrical surface 22a of the second cavity 22 comprises an abutment surface 22c configured to interact with an abutment surface 30a of the abutment element 30 in the working position of the nail 100. With reference now to the abutment element 30, the same is shown as a substantially axisymmetric body.

The abutment element 30 is preferably configured for being operationally disposed within the positioning body 20, thereby coming to be arranged, at least partially, within the first cavity 21 and/or second cavity 22 and coaxially with respect to the longitudinal axis "X".

The abutment element 30 is preferably movable relative to the positioning body 20 via a translatory movement, along a direction "T" coinciding with the longitudinal axis "X".

The abutment element 30 exhibits a first portion 31 so shaped as to abut against a head portion 110 of the nail 100 and a second portion 32 opposite the first portion 31. Preferably, the second portion 32 of the abutment element 30 has a cross section which is greater than a cross section of the first portion 31.

The first portion 31 of the abutment 30 has a protrusion 31 a so shaped as to abut against a notch or recess 150 of the head portion 110 of the nail 100.

Preferably, the protrusion 31 a is rib-shaped, still more preferably it is a protrusion of a circular shape.

The protrusion 31 a is so shaped as to be inserted, partially or completely, into a respective notch or recess 150 of the head portion 110 of the nail 100, so that the axis of the nail 100 is maintained in a position parallel to the longitudinal axis "X".

Additionally, the protrusion 31 a is able to prevent rotation of the nail 100 about its axis when said nail 100 passes from the rest position to the working position thereof. By way of a non-limiting example, on the head portion 110 of the nail 100, the notch or recess 150 may exhibit a transverse notched shape, like the one of a customary screw exhibiting a straight notched head, alternatively it may have a star-shaped recess or a recess of the Torx type. In accordance with a preferred embodiment of the present invention, the head portion 110 of the nail 100 has a recess 150 defined by a cylindrical surface and a bottom surface, preferably conical (as illustrated in the appended figures).

According to an alternative embodiment, the notch 150 is of the transverse type, defined by three flat surfaces which are orthogonal one to another, and adapted to receive a respective rib-shaped portion of the protrusion 31 a (this embodiment is not illustrated in the appended figures).

In particular, the abutment element 30 exhibits a contact surface 31 b at the first portion 31, which contact surface 31 b is configured to abut and match with a respective portion of a head surface 120 of the nail 100. Preferably, the head surface 120 of the nail 100 has a convex shape or otherwise shaped with a convex curvature.

As previously described, the abutment element 30 is movable relative to the positioning body 20 and in particular the abutment element 30 comprises a surface 32a relative to the second portion 32, which surface 32a is predisposed for receiving an impulsive force so as to generate a translation of the abutment element 30, that is, a passing from the rest position to the working position of the nail 100.

In detail, in the rest position of the nail 100 (shown schematically in Figure 1 attached hereto), the positioning body 20 of the head 10 is suitable to hold a nail 100 within the first recess 21, while the abutment element 30 is disposed rearward of the annular portion 23 and within the positioning body 20, thereby coming to occupy at least partially the second cavity 22. The protrusion 31 a of the abutment element is preferably already inserted within the notch or recess 150 on the head portion 110 of the nail 100. When the nail 100 is in the working position thereof, (illustrated in the appended Figure 2), the abutment element 30 abuts against the contact surface 31 b by translating along the translation direction "T", until it matches with a respective portion of the surface of head 120. At same time, the protuberance 31 a acts within the notch or recess 150 of the head portion 110 by pushing and guiding the nail 100 in the same direction "T" and according to the same translation condition of the abutment element 30. Similarly, the positioning body 20 is suitable to guide the nail 100, thus providing to keep said nail 100 aligned with respect to the translation direction "T" of the abutment element 30, so that proper insertion of the nail 100 into a portion of the bone tissue may occur.

In addition, during translation of the abutment element 30 from the second cavity 22 towards the first cavity 21, the abutment element 30 preferably penetrates further into the second cavity 22, thereby coming to be completely arranged therein.

Therefore, passing of the nail 100 of the head 10 from the rest position to the working position thereof, occurs owing to a translation of the abutment element 30 along the translation direction "T".

In accordance with the inventive concept of the present invention, the translation of the abutment element 30 can be determined by an impulsive force of the manual type, to be exerted by the surgeon or delivered by a tool, which tool is separate from the head 10 holding the nail 100, the impulsive force being directed from the second portion 32 up to the end surface 120 of the nail 100.

The abutment element 30 terminates translation thereof as soon as the abutment surface 30a goes to strike onto respective abutment surface 22c at the second cavity 22. The contact between the abutment surface 30a and the abutment surface 22c determines the foremost position of the abutment element 30 following translation from the second cavity 22 towards the first cavity 21, and thus it determines the foremost and outmost position of the nail 100 which is housed internally the first cavity 21.

According to the present invention, the rearmost position of the abutment element 30 relative to the positioning body 20 is the one which is furthest from the annular portion 23, similarly the abutment surface 30a is separate from and not in contact with the abutment surface 22c of the positioning body 20, as shown by way of example in the annexed Figure 1.

The foremost position of the abutment element 30 relative to the positioning body 20, is the one wherein the abutment surface 30a comes to be abutted against the abutment surface 22c of the positioning body 20, as illustrated by way of example in the appended Figure 2.

Preferably, between the rest position and the working position of the nail 100 of the head 10 for holding the nail 100, the abutment surface 30a is arranged between the first cavity 21 and second cavity 22 of the positioning body 20. By way of example, the translation of the abutment element 30 along the translation direction "T" is equal to about 3 mm. Advantageously, the mechanical containment/holding of the nail 100 which occurs through the annular portion 23 of the positioning body 20, allows a user to position the head 10 for holding a nail 100 at a pre-established insertion point, without the nail coming to be moved and/or misaligned with respect to the longitudinal axis "X". Following full insertion of the nail 100 into the deepest bone tissue, the annular portion 145 in relief advantageously allows to keep it in position, thereby coming an opposing force to be generated with respect to the bone pulp, such that extraction of the nail 100 therefrom is prevented.

The positioning body 20 preferably exhibits securing means relative to the second cavity 22, which securing means is arranged to reversibly fit the head 10 for holding the nail 100 to a respective portion 300 of a micro nails application device 200.

By way of example, the head 10 for holding the nail 100 can be made of a plastic material, but it may further comprise metal parts or whatsoever, without this having to be construed as limiting or reducing the functional aspect of the head 10 for holding a nail 100 in any manner. Preferably, the nail 100 is made of a metallic material and processed via machine tools suitable for working mechanical parts which are rather small in size.

By way of a non-limiting example, Figure 3 schematically illustrates a kit of a head 10 for holding a nail 100 for a device 200 of a nail bone implant applications in accordance with the inventive concept of the present invention.

Preferably, the kit of a head 10 for holding a nail 100 destined to a device 200 for nail applications, comprises:
- a head 10 for holding a nail as above described;
- a nail 100 for bone implantology arranged internally the head 10 and comprising a head portion 110 suitable for abutting against a contact surface 31 b of the abutment element 30 of the head 10 for holding a nail 100.

In particular, the nail 100 has a notch or recess 150 on the head portion 110, which notch or recess is suitable for housing a protrusion 31 a of the abutment element 30.

Advantageously, the kit of the head 10 for holding a nail 100 comprises the nail 100 already aligned and positioned, which nail is retained by a positioning body 20 of the kit of the head 10. The kit of the head 10 for holding a nail 100 may be packaged with a nail 100 having different sizes, so that the surgeon can select the kit which is best suited to that specific kind of application. Via the securing means 24 arranged on the positioning body 20, the kit of the head 10 for holding a nail 100 can be reversibly attached by the surgeon to a portion 300 of the device 200, for example it can be screwed or snap inserted, according to the securing means 24, which can be of the threaded or grafting type. In particular, after use, the kit of the head 10 for holding a nail 100 is removable owing to the securing means, wherein the housing portion 300 of the device 200 is left free thereof, for example for the purpose of mounting another kit of the head 10 for holding the nail 100.

It should be appreciated that, when the nail 100 of the kit of the head 10 is in the rest position, the nail 100 is retained within the first cavity 21 of the positioning body 20 for the whole length thereof. In other words, until an impulsive force is imparted to the abutment element 30 of the head 10, the nail 100, in the rest position thereof, does not protrude from the positioning body 20 even when already secured to a portion 300 of the device 200.

Indeed, when the nail 100 is in the rest position thereof, the head 10 of the kit is such as to completely incorporate the nail 100, thereby allowing the user to safely control and easily position the head 10 for holding a nail 100. Moreover, in this way the nail 100 is preserved sterile. The protuberance 31 a of the abutment element 30 is preferably inserted within a respective notch or recess 150 of the head portion 110 of the nail 100 in order to guide and maintain it axially aligned in a translation direction "T" of the abutment element 30.

In the working position of the nail 100 of the head 10, the nail 100 is guided and kept aligned with respect to the translation direction "T" of the abutment element 30 owning to the translation of said abutment element 30, which translation is induced by an impulsive force imparted by the device 200 for application of a nail, for example through an elastic system or a system of a different type which is part of above device 200.

In addition, the contact surface 31 b is configured to abut against and match with the respective portion of the surface of the head 120 of the nail 100 when the impulsive force is being transmitted from said abutment element 30 to the nail 100. In this way, a steadier positioning and alignment of the nail 100 is attainable than the one which can be obtained just inserting the protrusion 31 a into the respective notch or recess 150. Advantageously, the kit of the head 10 for holding a nail 100 comprises a sterile package such as to wrap and protect at least the head 10 for holding a nail 100 and a nail 100 for bone implant applications from the external atmosphere, in particular the nail 100 is already inserted and retained within the positioning body 20 for the whole length thereof, without protruding therefrom.

An identification code preferably appears on a portion of the sterile packaging of the kit of the head 10 for holding a nail 100, which code allows the surgeon to individuate, in a simple and safe manner, the type of nail 100 housed inside the head 10 which is not entirely visible, in this way surgeon's operations are facilitated and his/her work to complete the surgical intervention speeded up. The material whereof the kit of the head 10 for holding a nail 100 is made, is preferably a recyclable and low cost material, so that the kit is cost-effective, simple and with a low environmental impact.

The present invention attains the set aims thereby overcoming the drawbacks mentioned above.

Advantageously, the nail according to the present invention exhibits a sturdy structure which is at the same time simple and characterized by low production costs, and wherein a symmetrical recess which is arranged coaxially to the nail axis, confers the latter a very simple overall geometry. A further advantage of the inventive nail, is the effective insertion and position maintenance following insertion thereof into the bone tissue, despite lack of a threaded portion along its stem. Additionally, the simple geometry of the nail helps to reduce waste material during processing thereof and is thus furtherly cost-saving.

The kit of the head for holding a nail for bone implant applications enables a nail to be advantageously retained in a safe and sterile manner, thus allowing a safe insertion thereof by way of a simple and fast process. Advantageously, the head for holding a nail notably reduces the risk of error which may be due to a wrong or precarious positioning of the nail as known in the prior art.

Additionally, surgical operations to be performed by the surgeon is fewer in number than the prior art, thereby becoming workload as well as duration of the intervention reduced.

Advantageously, the head for holding a nail and the kit of the head for holding a nail according to the invention, helped to reduce stress generated by such a medical practice on the part of the surgeon, wherein repeteability of the surgical operation is increased.

Advantageously, the head for holding a nail is realized in a simple way, and with cost-effective materials allowing use of disposable kits, wherein, once the nail has been positioned and inserted into the bone tissue, the parts of the head for holding a nail can be easily removed from the device and sent to disposal as provided in the healthcare sector. Indeed it should be noted that, within the kit of the head for holding a nail, the nail itself is mainly the finest and most expensive component, whereas the support elements, such as the positioning body and the abutment element comprised therein, are purposely manufactured in such a manner that a considerable cost reduction is obtained in that, due to hygienic and practical reasons, said parts can no longer be reused.

## Claims

1. A head (10) for holding a nail (100) for bone implant applications, **characterized in that** it comprises:
- a positioning body (20) having a first cavity (21) suitable for reversibly holding a nail (100) and a second cavity (22) opposite to and in communication with said first cavity (21) and wherein said first cavity (21) and said second cavity (22) are open outwardly;
- an abutment element (30) associated with said positioning body (20) and configured to interact with said nail (100) to bring it from a rest position to an working position;
wherein said positioning body (20) is suitable for holding said nail (100) in said first cavity (21) in said rest position and wherein said positioning body (20) is configured to control said nail (100) and keeping it aligned with respect to a direction of translation (T) of said abutment element (30) when passing from the rest position to the working position of the nail (100).

2. A head (10) for holding a nail (100) according to claim 1, wherein said abutment element (30) is arranged inside said positioning body (20) occupying at least partially said first cavity (21) and/or said second cavity (22) of said positioning body (20).

3. A head (10) for holding a nail (100) according to claim 1, wherein said positioning body (20) has said first cavity (21) and said second cavity (22) aligned along a longitudinal axis (X), said longitudinal axis (X) being preferably the main axis of development of said first and second cavity (21, 22).

4. A head (10) for holding a nail (100) according to claim 3, wherein said direction of translation (T) of said abutment element (30) is coaxial with said longitudinal axis (X).

5. A head (10) for holding a nail (100) according to claim 1, wherein said first cavity (21) of said positioning body (20) has a smaller cross section than the cross section of said second cavity (22) of said positioning body (20).

6. A head (10) for holding a nail (100) according to claim 5, wherein said abutment element (30) has a first portion (31) so shaped as to abut against a head portion (110) of said nail (100) and a second portion (32) having a larger cross section compared to a cross section of said first portion (31).

7. A head (10) for holding a nail (100) according to claim 6, wherein said first portion (31) of said abutment element (30) has a protrusion (31 a) shaped to fit into a notch or recess (150) of said head portion (110) of said nail (100), said protrusion (31 a) being preferably rib-shaped.

8. A head (10) for holding a nail (100) according to claim 6, wherein said abutment element (30) exhibits a contact surface (31 b) at said first portion (31), said contact surface (31 b) being configured to abut against and match with a respective portion of a head surface (120) of said nail (100).

9. A head (10) for holding a nail (100) according to claim 1, wherein said abutment element (30) has a first portion (31) so shaped as to abut against a head portion (110) of said nail (100) and/or a contact surface (31 b) configured to abut against and match with a respective portion of a head surface (120) of said nail (100).

10. A head (10) for holding a nail (100) according to any one of the preceding claims 6-9, wherein said second portion (32) of said abutment element (30) comprises a surface (32a) arranged to receive an impulsive force to generate a translation of said abutment element (30).

11. A head (10) for holding a nail (100) according to any one of the preceding claims, wherein said first cavity (21) of said positioning body (20) is defined by a cylindrical surface (21 a) having a diameter (d) compatible with the maximum diameter of said head surface (120) of said nail (100).

12. A head (10) for holding a nail (100) according to claim 11, wherein said positioning body (20) comprises an annular portion (23) arranged at one open end of said first cavity (21) and having radial notches (23b), said annular portion (23) preferably having an internal diameter approximately equal to the diameter (d) of said surface of cylindrical shape (21 a).

13. A head (10) for holding a nail (100) according to any one of the preceding claims, wherein said positioning body (20) has on one bottom end of said second cavity (22) an annular-shaped abutment surface (22b) suitable for receiving a respective abutment surface (31 b) of said abutment element (30), said abutment surface (22b) being preferably arranged between said first portion (21) and said second portion (22) of said positioning body (20).

14. A head (10) for holding a nail (100) according to one or more of the preceding claims, wherein said positioning body (20) comprises at said the second cavity (22), securing means (24) for reversibly fitting said head (10) for holding nails (100) onto a portion of a device (200) for applying said nail, said securing means (24) being preferably of the threaded type.

15. A nail (100) for bone implant applications comprising a head portion (110), a stem (130) and a terminal sharp portion (140) and wherein an annular portion in relief (145) is preferably comprised between said terminal sharp portion (140) and said stem (130).

16. A nail (100) according to claim 15, wherein the head portion (110) has a notch or recess (150) defined by a cylindrical surface and a preferably conical bottom surface.

17. A kit for a device (200) for applying a nail (100) for bone implant comprising:
- a head (10) for holding a nail (100) according to claim 14;
- a nail (100) for bone implants according to claim 16 arranged within said head (10) and wherein said head portion (110) is suitable for abutting against a contact surface (31 b) of the abutment element (30).

18. A kit according to claim 17, wherein said nail (100) is placed in said head (10) in said rest position such that said nail (100) is retained in said positioning body (20) for its entire length.

19. A kit according to claim 18, wherein said nail (100) has a notch or recess (150) on said head portion (110), which notch or recess (150) is suitable for receiving a protrusion (31 a) of said abutment element (30) and preferably wherein said contact surface (31 b) is configured to abut against and match with a respective surface (120) of said head portion (110) of said nail (100).

20. A kit according to one or more of claims 16-19, comprising a sterile package such as to wrap and protect from the external atmosphere at least said head (10) for holding a nail (100) and said nail (100) for bone implants.
